# NEUE EUROPÄISCHE PATENTSCHRIFT

(11) **EP 1 598 064 B2**
(45) Veröffentlichungstag und Bekanntmachung des Hinweises auf die Entscheidung über den Einspruch: **25.02.2015**
(45) Hinweis auf die Patenterteilung: 09.11.2011
(21) Anmeldenummer: 04021126.0
(22) Anmeldetag: 06.09.2004
(51) Int. Cl.: A61K 31/19, A61K 8/365, A61K 31/047, A61K 8/34, A61P 17/10

(54) **Kombination von Alkandiol und Hydroxysäure zur Behandlung von Akne**
Combination of alkane diol and hydroxy acid for the treatment of acne
Combination de alcane-diol de hydroxy-acide pour le traitement de l'acne

(30) Priorität: 06.09.2003 DE 10341179
(43) Veröffentlichungstag der Anmeldung: 23.11.2005
(73) Patentinhaber: Symrise AG, 37603 Holzminden (DE)
(72) Erfinder: Traupe, Bernd, 22457 Hamburg (DE); Fänger, Sabine, 21147 Hamburg (DE); Filbry, Alexander, 22459 Hamburg (DE); Bürger, Anette, 22301 Hamburg (DE); Jung, Susanne, 22529 Hamburg (DE)
(74) Vertreter: Eisenführ Speiser

(56) Entgegenhaltungen:
- EP-A- 0 721 775
- EP-A2- 0 043 738
- EP-A2- 0 086 070
- EP-A2- 0 584 692
- WO-A1-89/00853
- WO-A1-96/11572
- WO-A1-96/27365
- DE-A- 10 148 302
- DE-A1- 3 815 574
- DE-A1- 4 225 985
- FR-A1- 2 747 572
- JP-A- H11 322 591
- US-A- 4 507 319
- US-A- 4 954 487
- US-A- 6 123 953
- US-A1- 2002 098 220
- US-B1- 6 290 937

## Beschreibung

Die vorliegende Erfindung betrifft eine Wirkstoffkombination aus α-und/oder ß-Hydroxysäuren und Alkan-1,2-diolen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Einen wesentlichen Anteil an einem schönen und attraktiven Äußeren hat dabei der Zustand und das Aussehen der Haut.

Bei fettig-öliger und unreiner Haut, als welche man den Übergangszustand zwischen der gesunden normalen und der krankhaft veränderten Aknehaut bezeichnet, produziert die Haut erhöhte Mengen an Talg (Seborrhoe). Dieser dient zahlreichen Mikroorganismen, insbesondere *Propionibacterium acnes* und *Pityrosporum-Arten* als idealer Nährboden. Die Mikroorganismen zersetzen den Talg zu Glycerin und Fettsäuren, wodurch die Talgdrüsen zu erhöhter Produktion angeregt und die Folliketwandungen in der Haut angegriffen und zerstört werden. Dies ruft Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird (W. Umbach [Hrsg], Kosmetik, Entwicklung, Herstellung und Anwendung kosmetischer Mittel, 2.Aufl. Thieme Verlag, Stuttgart, 1995).

Unter Akne (im engeren Sinne *acne vulgaris*) *versteht* man verschiedene Erkrankungen der Talgdrüsenfollikel, die durch Sekretions- und Verhornungsstörungen nachfolgende Entzündungen und eventuelle Vemarbung gekennzeichnet sind. Die *acne vulgaris tritt vor* hauptsächlich in der Pubertät auf und konzentriert sich in der Regel auf talgdrüsenreiche Hautbezirke (Gesicht, Nacken, Brust, Rücken). Durch Talgdrüsenhyperplasie und eine Verhornungsstörung der Follikel kommt es zu deren Verstopfung mit Bildung von Komedonen und den für *acne* vulgaristypischen Effloreszenzen (Pschyrembel, Klinisches Wörterbuch, 258. Aufl., Walter de Gruyter-Verlag, Berlin, 1998).

Herkömmliche Produkte zur Behandlung fettiger und/oder unreiner Haut haben in der Regel den Nachteil die Haut zu strapazieren, auszutrocknen und wenig pflegend zu wirken. Bei der Behandlung der Akne werden darüber hinaus stark saure (z.B. Milchsäure bei pH-Werten kleiner pH 4,0) sowie stark oxidierende Wirkstoffe (z.B. Benzoylperoxid) eingesetzt, welche den Säureschutzmantel der Haut strapazieren und die Haut angreifen bzw. verätzen.

Es war daher die Aufgabe der vorliegenden Erfindung eine deutlich mildere und besser pflegende kosmetische und/oder dermatologische Zubereitung zur Behandlung fettiger und unreiner Haut sowie zur Behandlung von Akne (und insbesondere *acne vulgaris*) zu entwickeln, als sie vom Stand der Technik her bekannt sind. Ferner war es die Aufgabe der vorliegenden Erfindung, eine kosmetische und/oder dermatologische Zubereitung zur Behandlung von Akne (und insbesondere *acne vulgaris*) zu entwickeln, die bei einem hautfreundlichen pH-Wert (pH grösser pH 4,0) wirksam ist, die Haut nicht verätzt und den Säureschutzmantel der Haut allenfalls gering belastet. Es sollten ferner Zubereitungen entwickelt werden, die bei einem hautfreundlichen pH-Wert (pH grösser pH 4,0) das Wachstum und die Verbreitung von Akne hervorrufenden Bakterien auf der Haut, insbesondere von *Propionibakterium acnes* unterdrücken. Ferner sollte die Konzentration an Akne hervorrufenden Bakterien auf der Haut und in den Talgdrüsen durch diese Zubereitungen signifikant reduziert werden.

Überraschend gelöst wird die Aufgabe durch eine kosmetische und/oder dermatologische Zubereitung oder Verwendung einer solchen Zubereitung jeweils wie in den Ansprüchen beschrieben.

Zwar ist die therapeutische Wirkung von α-und/oder β-Hydroxysäuren bei Akne an sich bekannt, doch werden die herkömmlichen Zubereitungen bei einem pH-Wert von kleiner 4,0 eingesetzt.

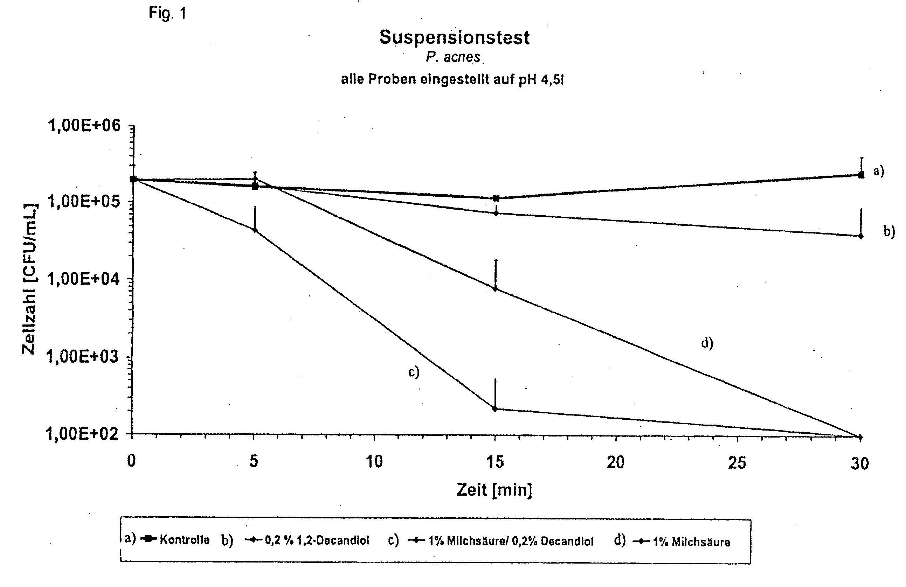

Auch ist die antibakterielle Wirkung von Alkan-1,2-diolen an sich bekannt. So beschreibt die JP 20022003330 die antibakterielle Wirkung einer Kombination von 1,2-Alkandiolen und Ascorbinsäureestem. Die WO 03/000220 beschreibt die Verwendung von 1,2-Decandiol gegen Körpergeruch verursachende Bakterien. Nicht zuletzt beschreibt die US 6,123,953 die Verwendung von Alkan-1,2-diolen zur Inaktivierung von Mikroorganismen auf der Haut durch den Einsatz von 1,2-Octandiol. In dieser Schrift wird auch die Wirkung von **1,2-Octandiol** gegen Akne erwähnt. Doch konnte diese Schrift nicht den Weg zur vorliegenden Erfindung weisen, denn überraschend und für den Fachmann nicht vorhersehbar ist die synergistische Wechselwirkung von α-und/oder β-Hydroxysäuren (insbesondere Milchsäure) Alkan-1,2-diolen (insbesondere 1,2-Decandiol (= Decan-1,2-diol)), die unter anderem aus dem Vergleichsversuch (Fig. 1) ersichtlich ist.

Selbst für den Fachmann überraschend war ferner der Umstand, dass die erfindungsgemäße Zubereitung schneller und länger anhaltend wirksam ist als Zubereitungen, die lediglich einen der Wirkstoffe enthalten. Die erfindungsgemäße Zubereitung weist damit eine deutliche und nicht vorhersehbare synergistische Wechselwirkung zwischen einerseits α-und/oder β-Hydroxysäuren (insbesondere Milchsäure) sowie andererseits Alkan-1,2-diolen (insbesondere Decan-1,2-diol) auf.

Die zu verwendenden Wirkstoffkombinationen werden insbesondere erfindungsgemäß vorteilhaft in kosmetischen und/oder dermatologischen Zubereitungen verwendet.

Die erfindungsgemäßen Zubereitungen sind dadurch gekennzeichnet, dass der pH-Wert der Zubereitung größer als pH 4,5 ist. Dabei sollte der pH-Wert einen Wert von pH 7,5 und besonders einen pH-Wert von pH 7,0 nicht übersteigen.

Es ist erfindungsgemäß von Vorteil, wenn die erfindungsgemäßen Zubereitungen, dadurch gekennzeichnet sind, dass als α-und/oder β-Hydroxysäure eine α-Hydroxysäure eingesetzt wird. Erfindungsgemäß bevorzugt ist dabei der Einsatz von Milchsäure.

Es ist erfindungsgemäß von Vorteil, wenn die erfindungsgemäßen Zubereitungen, dadurch gekennzeichnet sind, dass als Alkan-1,2-diol Decan-1,2-diol eingesetzt wird.

Es ist erfindungsgemäß von Vorteil, wenn in den erfindungsgemäßen Zubereitungen, ein oder mehrere α-und/oder β-Hydroxysäuren in einer Gesamtkonzentration von 0,1 bis 10 Gewichts-%, bevorzugt in einer Konzentration von 0,2 bis 5 Gewichts-% und ganz besonders bevorzugt in einer Konzentration von 0,5 bis 4 Gewichts-%, jeweils bezogen auf das Gesamtgewicht der Zubereitung eingesetzt werden.

Es ist erfindungsgemäß von Vorteil, wenn in den erfindungsgemäßen Zubereitungen ein oder mehreren Alkan-1,2-diole in einer Gesamtkonzentration von 0,1 bis 5 Gewichts-%, bevorzugt in einer Konzentration von 0,3 bis 3 Gewichts-% und ganz besonders bevorzugt in einer Konzentration von 0,5 bis 2 Gewichts-%, jeweils bezogen auf das Gesamtgewicht der Zubereitung eingesetzt werden.

Darüber hinaus können Zubereitungen gemäß der vorliegenden Erfindung weitere bakteriozide und/oder bakteriostatische Wirkstoffe enthalten, wie beispielsweise Benzoylperoxid und/oder Schwefel. Auch Chlorhexidin, Triclosan, Bisabolol, Farnesol und Phenoxyethanol sowie Isoflavonoide (z.B. Genistein, Daidzein, Genistin und Glycitein) können erfindungsgemäß vorteilhaft mit der erfindungsgemäßen Wirkstoffkombination kombiniert werden.

Erfindungsgemäß vorteilhaft kann die erfindungsgemäße (dies beinhaltet auch immer die erfindungsgemäß verwendete) Wirkstoffkombination verkapselt werden. Dies kann beispielsweise durch den Einsatz von einem oder mehreren Cyclodextrinen und/oder deren Derivaten (z.B. gewählt aus der Gruppe α-Cyclodextrin, β-Cyclodextrin, Hy/droxypropyl-β-Cyclodextrin, methyliertes β-Cyclodextrin (random-Methyl-β-Cyclodextrin), rCyclodextrin) erfolgen. Auch der Einsatz von Lipid- und/oder Wachspartikeln zur Verkapselung ist erfindungsgemäß vorteilhaft.

Die erfindungsgemäßen Zubereitungen, können in verschiedenen Formen vorliegen. So können sie z. B. eine Lösung (z.B. wässrige, wässrig-alkoholische oder alkoholische Lösung), eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O) (jeweils auch in Form von Silikonöl-Emulsionen), eine Hydrodispersion o der L ipodispersion, e ine P ickering-Emulsion, e inen festen S tift o der a uch e in A erosol darstellen. Auch ist die Verwendung einer solchen erfindungsgemäßen Zubereitung als Tränkung für ein wasserunlösliches Substrat (z.B. ein Tuch , ein Vlies, ein Pad) erfindungsgemäß vorteilhaft, wobei es sich bei dieser Applikationsform sowohl um ein trocken anmutendes als auch um ein feucht anmutendes Substrat handeln kann.

Erfindungsgemäß vorteilhafte Darreichungsformen der erfindungsgemäßen Zubereitungen sind Cremes, Salben, Hydrodispersionen, Lotionen, Tinkturen, Pumpsprays, Aerosolsprays, wässrige Lösungen, Reinigungssubstrate und dergleichen.

Die wässrige Phase der erfindungsgemäßen Zubereitung kann neben Wasser erfindungsgemäß a uch andere Inhaltsstoffe e nthalten, beispielsweise Alkohole, D iole o der Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyloder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die erfindungsgemäße Zubereitung kann erfindungsgemäß vorteilhaft eine oder mehrere Öl-Lipid- und/oder Fettphasen enthalten. Diese kann beispielsweise aus den folgenden Verbindungen gebildet werden:

Polare Öle, beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe d er s ynthetischen, h albsynthetischen u nd n atürlichen Öle, w ie z.B. O livenöl, S onnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Hersteller** | **Handelsname** | **INCI-Name** | **Polarität [mN/m]** |
|---|---|---|---|
| Condea Chemie | Isofol 14 T | Butyl Decanol (+) Hexyl Octanol (+) Hexyl Decanol (+) Butyl Octanol | 19,8 |
| Lipochemicals INC. / USA (Induchem) | Lipovol MOS-130 | Tridecyl.Stearate(+) Tridecyl Trimellitate(+) Dipentaerythrityl Hexacaprylate/Hexacaprate | 19,4 |
| | Ricinusoel | | 19,2 |
| CONDEA Chemie | Isofol Ester 0604 | | 19,1 |
| Huels CONDEA Chemie | Miglyol 840 | Propylene Glycol Dicaprylate/Dicaprate | 18,7 |
| *CONDEA Chemie* | Isofol 12 | Butyl Octanol | 17,4 |
| Goldschmidt | Tegosoft SH | Stearyl Heptanoate | 17,8 |
| | Avocadooel | | 14,5 |
| Henkel Cognis | Cetiol B | Dibutyl Adipate | 14,3 |
| ALZO (ROVI) | Dermol 488 | PEG 2 Diethylenhexanoate | 10,1 |
| Condea Augusta S.P.A. | Cosmacol ELI . | C12-13 Alkyl Lactate | 8,8 |
| ALZO (ROVI) | Dermol 489 | Diethylen Glycol Dioctanoate(/ Diisononanoate | 8,6 |
| Condea Augusta S.P.A. | Cosmacol ETI | Di-C12/13 Alkyl Tartrate | 7,1 |
| Henkel Cognis | Emerest 2384 | Propylene Glycol Monoisostearate | 6,2 |
| Henkel Cognis | Myritol 331 | Cocoglycerides | 5,1 |
| Unichema | Prisorine 2041 GTIS | Triisostearin | 2,4 |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole s ind b enannt n ach M arcel G uerbet, d er i hre H erstellung e rstmalig b e-schrieb. Sie entstehen nach der Reaktionsgleichung 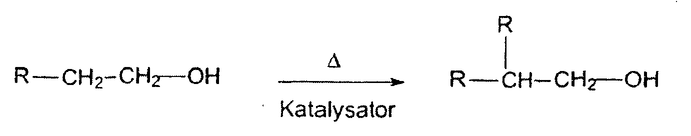 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 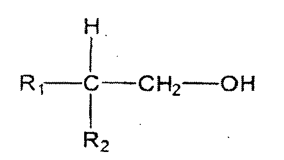 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 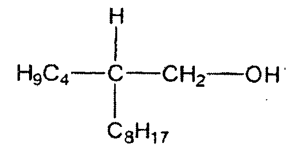 und ist beispielsweise unter der Handelsbezeichnung Isofol® 12 von der Gesellschaft Condea Chemie GmbH erhältlich - und das 2-Hexyldecanol - es hat die chemische Struktur 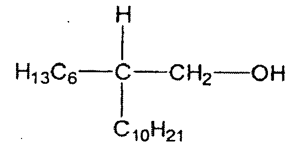 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und-Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

| **Hersteller** | **Handelsname** | **INCI-Name** | **Polarität [mN/m]** |
|---|---|---|---|
| Stearinerie Dubois Fils | DUB VCI 10 | Isodecyl Neopentanoate | 29,9 |
| ALZO (ROVI) | Dermol IHD | Isohexyldecanoate | 29,7 |
| ALZO (ROVI) | Dermol 108 | Isodecyl Octanoate | 29,6 |
| | Dihexyl Ether | Dihexyl Ether | 29,2 |
| ALZO (ROVI) | Dermol 109 | Isodecyl 3,5,5 Trimethyl Hexanoate | 29,1 |
| Henkel Cognis | Cetiol SN | Cetearyl Isononanoate | 28,6 |
| Unichema | Isopropylpalmitat | Isopropylpalmitat | 28,8 |
| Dow Corning | DC Fluid 345 | Cyclomethicone | 28,5 |
| Dow Corning | Dow Corning Fluid 244 | Cyclopolydimethylsiloxan | 28,5 |
| Nikko Chemicals Superior Jojoba Oil Gold | Jojobaöl Gold | | 26,2 |
| Wacker | Wacker AK 100 | Dimethicone | 26,9 |
| ALZO (ROVI) | Dermol 98 | 2- Ethylhexanosäure 3,5,5 Trimethylester | 26,2 |
| Dow Corning | Dow Corning Fluid 246 | Offen | 25,3 |
| Henkel Cognis | Eutanol G | Octyldodecanol | 24,8 |
| Condea Chemie | Isofol 16 | Hexyl Decanol | 24,3 |
| ALZO (ROVI) | Dermol 139 | Isotridecyl 3,5,5 Trimethylhexanonanoate | 24,5 |
| Henkel Cognis | *Cetiol PGL* | Hexyldecanol (+) Hexyl Decyl Laurate | 24,3 |
| | Cegesoft C24 | Octyl Palmitate | 23,1 |
| Gattefossé | M.O.D. | Octyldodeceyl Myristate | 22,1 |
| | Macadamia Nut Oil | | 22,1 |
| Bayer AG, Dow Corning | *Silikonöl VP 1120* | Phenyl Trimethicone | 22,7 |
| CONDEA Chemie | Isocarb 12 | Butyl Octanoicacid | 22,1 |
| Henkel Cognis | Isopropylstearat | Isopropyl Stearate | 21,9 |
| WITCO, Goldschmidt | Finsolv TN | C12-15 Alkyl Benzoate | 21,8 |
| Dr. Straetmans | Dermofeel BGC | Butylene Glycol Caprylate/Caprate | 21,5 |
| Unichema Huels | Miglyol 812 | Caprylic/Capric Triglyceride | 21,3 |
| Trivent (über S. Black) | Trivent OCG | Tricaprylin | 20,2 |
| ALZO (ROVI) | Dermol 866 | PEG" Diethylhexanoate/ Diisononanoate/ Ethylhexyl Isononanoate | 20,1 |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Hersteller** | **Handelsname** | **INCI-Name** | **Polarität [mN/m ]** |
|---|---|---|---|
| Total SA | Ecolane 130 | Cycloparaffin | 49,1 |
| Neste PAO N.V. (Lief. Hansen & Rosenthal) | Nexbase 2006 FG | Polydecene | 46.7 |
| Chemische Fabrik Lehrte | Polysynlane | Hydrogenated Polyisobutene | 44.7 |
| Wacker | Wacker Silikonöl AK 50 | Polydimethylsiloxan | 46,5 |
| EC Erdölchemie (Lieferant Bayer AG) | Solvent ICH | Isohexadecane | 43.8 |
| DEA Mineralöl (Lief. Hansen & Rosenthal) Tudapetrol | Pionier 2076 | Mineral Oil | 43.7 |
| DEA Mineralöl (Lief. Hansen & Rosenthal) Tudapetrol | Pionier 6301 | Mineral Oil | 43.7 |
| Wacker | Wacker Silikonöl AK 35 | Polydimethylsiloxan | 42,4 |
| EC Erdölchemie GmbH | Isoeikosan | Isoeikosan | 41.9 |
| Wacker | Wacker Silikonöl AK 20 | Polydimethylsiloxan | 40,9 |
| Condea Chemie | Isofol 1212 Carbonat | | 40,3 |
| Gattefosse | Softcutol O | Ethoxydiglycol Oleate | 40,5 |
| Creaderm | Lipodermanol OL | Decyl Olivate | 40,3 |
| *Henkel* | *Cetiol S* | Dioctylcyclohexane | 39,0 |
| DEA Mineralöl (Lief. Hansen & Rosenthal) Tudapetrol | Pionier 2071 | Mineral Oil | 38.3 |
| WITCO BV | Hydrobrite 1000 PO | Paraffinum Liquidum | 37,6 |
| Goldschmidt | Tegosoft HP | Isocetyl Palmitate | 36,2 |
| Condea Chemie | Isofol Ester 1693 | | 33,5 |
| Condea Chemie | Isofol Ester 1260 | | 33,0 |
| Dow Corning | Dow Corning Fluid 245 | Cyclopentasiloxan | 32,3 |
| Unichema | Prisorine 2036 | Octyl Isostearate | 31.6 |
| Henkel Cognis | Cetiol CC | Dicaprylyl Carbonate | 31,7 |
| ALZO (ROVI) | Dermol 99 | Trimethylhexyl Isononanoate | 31,1 |
| ALZO (ROVI) | Dermol 89 | 2- Ethylhexyl Isononanoate | 31,0 |
| Henkel Cognis | Cetiol OE | Dicaprylyl Ether | 30,9 |
| | *Dihexylcarbonat* | Dihexyl Carbonate | 30,9 |
| Albemarle S.A. | Silkflo 366 NF | Polydecene | 30,1 |
| Unichema | Estol 1540 EHC | Octyl Cocoate | 30.0 |

Erfindungsgemäße Zubereitungen können auch vorteilhaft Verdicker enthalten. Geeignete Verdicker sind:
Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX.
Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum.
Außerdem geeignet sind Copolymere der Acrylsäure oder der Methacrylsäure wie z.B. Carbopol 1342 oder Permulen TRI.
Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

Die erfindungsgemäßen kosmetischen und/oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Zum anderen können erfindungsgemäß vorteilhaft in den Zubereitungen Antitranspirantien eingesetzt werden, welche die Schweißabsonderung durch Blockierung der Schweißdrüsenausgänge behindern. Verwendung finden hier in aller Regel Aluminium- und Aluminium/Zirkoniumsalze.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Dabei können erfindungsgemäß vorteilhaft die einzelnen Phasen der Zubereitung unterschiedlich gefärbt sein. Auch Ausführungsformen der Erfindung, bei denen lediglich eine der beiden Phasen gefärbt ist, sind erfindungsgemäß vorteilhaft. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe Colour Index, 3. Auflage, Society of Dyers and Colourists, Bradford, England, 1971 zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| | | |
|---|---|---|
| | | |
| | TiO₂: 40 - 60 nm | silber |
| | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der F irma M erck v ertrieben werden u nd s ich b esonders für d ie o ptische R eduktion feiner Fältchen eignen.

Es kann darüber hinaus von Vorteil sein, gänzlich auf ein Substrat wie Glimmer zu verzichten. B esonders b evorzugt s ind E isenperlglanzpigmente, w elche o hne d ie V erwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch in Kombination vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Auch können alle für Desodorantien gängigen Wirkstoffe vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein. Die Menge der Desodorantien (eine oder mehrere Verbindungen) in den Zubereitungen beträgt vorzugsweise 0,01 bis 10 Gew.%, besonders bevorzugt 0,05 bis 5 Gew.%, insbesondere 0,1 bis 1 Gew.%, bezogen auf das Gesamtgewicht der Zubereitung.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Antioxidantien, Parfüme, Hydrocolloide, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate sowie Moisturizer. Auch können sie kosmetische und/oder dermatologische Wirkstoffe enthalten, die sowohl öl- als auch wasserlöslich sein können.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Vorteilhafte anfeuchtende bzw. feuchthaltende Mittel (sogenannte Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen Zubereitungen können e rfindungsgemäß vorteilhaft in Form einer Reinigungszubereitung vorliegen. Insbesondere in einem solchen Falle enthält die erfindungsgemäße Zubereitung vorteilhaft ein oder mehrere oberflächenaktive Stoffe.

### A. Anionische Tenside

Vorteilhaft zu verwendende anionische Tenside sind
Acylaminosäuren (und deren Salze), wie
- 1.: Acylglutamate, beispielsweise Natriumacylglutamat, Di-TEA-palmitoylaspartat und Natrium Caprylic/ Capric Glutamat,
- 2.: Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
- 3.: Sarcosinate, beispielsweise Myristoyl Sarcosin, TEA-lauroyl Sarcosinat, Natriumlauroylsarcosinat und Natriumcocoylsarkosinat,
- 4.: Taurate, beispielsweise Natriumlauroyltaurat und Natriummethylcocoyltaurat,
- 5.: Acyllactylate, Lauroyllactylat, Caproyllactylat
- 6.: Alaninate
Carbonsäuren und Derivate, wie
- 1.: Carbonsäuren, beispielsweise Laurinsäure, Aluminiumstearat, Magnesiumalkanolat und Zinkundecylenat,
- 2.: Ester-Carbonsäuren, beispielsweise Calciumstearoyllactylat, Laureth-6-Citrat und Natrium PEG-4-Lauramidcarboxylat,
- 3.: Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
- 1.: Acyl-isethionate, z.B. Natrium-/ Ammoniumcocoyl-isethionat,
- 2.: Alkylarylsulfonate,
- 3.: Alkylsulfonate, b eispielsweise N atriumcocosmonoglyceridsulfat, N atrium C₁₂₋₁₄ O lefinsulfonat, Natriumlaurylsulfoacetat und Magnesium PEG-3 Cocamidsulfat,
- 4.: Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
- 1.: Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
- 2.: Alkylsulfate, beispielsweise Natrium-, Ammonium- und TEA-Laurylsulfat.

### B. Kationische Tenside

Vorteilhaft zu verwendende kationische Tenside sind
- 1.: Alkylamine,
- 2.: Alkylimidazole,
- 3.: Ethoxylierte Amine und
- 4.: Quaternäre Tenside.
- 5.: Esterquats

Quaternäre Tenside enthalten mindestens ein N-Atom, das mit 4 Alkyl- und/oder Arylgruppen kovalent verbunden ist. Dies führt, unabhängig vom pH W ert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryloder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
- 1.: Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
- 2.: N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
- 1.: Alkohole,
- 2.: Alkanolamide, wie Cocamide MEA/ DEA/ MIPA,
- 3.: Aminoxide, wie Cocoamidopropylaminoxid,
- 4.: Ester, die durch Veresterung von Carbonsäuren mit Ethylenoxid, Glycerin, Sorbitan oder anderen Alkoholen entstehen,
- 5.: Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
- 6.: Sucroseester, -Ether
- 7: Polyglycerinester, Diglycerinester, Monoglycerinester
- 8.: Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Insgesamt beträgt der Gehalt ein einem oder meherern Tensiden in den erfindungsgemäßen Zubereitungen 0,001 bis 30 Gewichts-%, bezogen auf das Gesamtgewicht der Zubereitung.

Erfindungsgemäß vorteilhaft können erfindungsgemäße Zubereitungen Polysorbate enthalten. Polysorbate stellen eine Verbindungsklasse dar, die sich vom Sorbitan, einem aus Sorbit durch Abspaltung zweier Äquivalente Wasser gewonnenem Furanderivat, ableiteten. Die Hydroxylgruppen des Sorbitans sind mit Polyethylenglykolen verethert, deren Enden mit Fettsäuren verestert sein können. Sie lassen sich allgemein durch die Formel 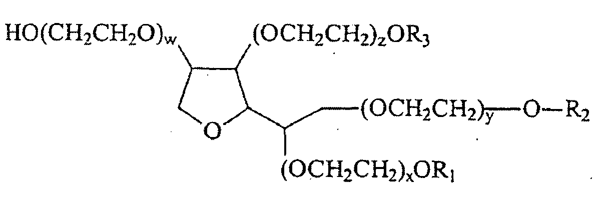 R₁, R₂, R₃ = H, Fettsäurerest
darstellen.

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Zubereitung einzeln oder als Mischung mehrer Polysorbate, eingesetzt.

Die kosmetischen und dermatologischen Zubereitungen gemäß der Erfindung können kosmetische Hilfsstoffe enthalten, wie sie üblicherweise in solchen Zubereitungen verwendet werden, z. B. Parfüme, Substanzen zum Verhindern oder Steigern des Schäumens, Verdickungsmittel, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate. Auch weitere Wirkstoffe wie beispielsweise Repellentien, Selbstbräuner, Depigmentierungsmittel, Pflanzenextrakte, Vitamine, können erfindungsgemäß vorteilhaft ein die erfindungsgemäßen Zubereitungen eingearbeitet werden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele 1-10: O/W-Cremes

| **Beispiel Nummer** | **1** | **2** | **3** | **4** | **5** |
|---|---|---|---|---|---|
| Glycerylstearatcitrat | 2 | | | 2 | |
| Glycerylstearat, selbstemulgierend | | 5 | 3 | | 2 |
| PEG-40-Stearat | | | 1 | | 1 |
| Myristylmyristat | 1 | | | | 1 |
| Stearylalkohol | 2 | 1 | | | |
| Cetearylalkohol | | | | 4 | 2 |
| Cetylalkohol | 1 | | 3 | | |
| Hydrierte Kokosfettglyceride | 2 | | | | |
| Sheabutter | | 2 | | | 2 |
| C12-15 Alkylbenzoat | | | 2 | | |
| Caprylsäure/Caprinsäure Triglycerid | | | 1 | | 2 |
| Ethylhexylkokosfettsäureester | 3 | | | | 1 |
| Octyldodecanol | | | 1 | | |
| Octamethyltetrasiloxan | | 1 | | 3 | |
| Dimethylpolysiloxan | | | 1 | 1 | |
| Dicaprylylether | 1 | 4 | 2 | | |
| Dicarprylylcarbonat | | | | 3 | |
| Ubichinon (Q10) | 0,05 | | | | |
| 1,2- Decandiol | 0,5 | 0,3 | | 0,3 | 0,5 |
| 1,2- Octandiol | | | 0,2 | | 0,5 |
| Salicylsäure | 0,5 | | 0,2 | 0,2 | 1,0 |
| Glycolsäure | | | | 1,0 | |
| Milchsäure | | 2,5 | 0,2 | 1,0 | |
| Citronensäure | | 0,5 | | | |

| **Beispiel Nummer** | **1** | **2** | **3** | **4** | **5** |
|---|---|---|---|---|---|
| | | | | | |
| Natriumascorbylphosphat | 0,1 | | | | |
| Trinatrium EDTA | | 0.1 | | 0,2 | |
| Iminodisuccinat, Natriumsalz | 0,2 | | 0,1 | | |
| Phenoxyethanol | 0,3 | | 0,3 | 0,2 | 0,2 |
| p-Hydroxybenzoesäurealkylester | 0,6 | 0,3 | 0,2 | 0,3 | 0,3 |
| Hexamidindiisethionat | | 0,04 | | | |
| Diazolidinylharnstoff | | | 0,1 | | |
| 1,3-Dimethylol-5,5-dimethylhydantoin | | 0,2 | | | |
| lodopropynylbutylcarbamat | | | | | 0,1 |
| Ethanol denaturiert | | 2 | | | |
| Xanthan Gummi | 0,1 | | | | |
| Polyacrylsäure (Carbomer) | 0,05 | | 0,1 | | 0,1 |
| Glycerin | 10 | 6 | 6 | 7,5 | 8 |
| Butylenglycol | 2 | 1 | | | |
| Wasser- und/oder öllösliche Farbstoffe | 0,05 | | | | |
| Füllstoffe/Additive (Distärkephosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Natriumhydroxid ad pH > 4,0 | q.s. | q.s. | q.s. | q.s. | q.s. |
| Wasser | Ad 100 | Ad 100 | Ad 100 | Ad 100 | Ad 100 |

| Beispiel Nummer | 6 | 7 | 8 | 9 | 10 |
|---|---|---|---|---|---|
| Glycerylsterat, selbstemulgierend | 2,5 | | | | |
| PEG-40-Stearat | 1 | | | | |
| Polyglyceryl-3-Methylglucosedistearat | | 3 | | | |
| Sorbitanstearat | | 1 | | | |
| Polyethylenglycol(21)stearylether | | | 2 | | |
| Polyethylenglycol(2)stearylether | | | 1 | | |
| Cetearylglucosid | | | | 2 | |
| Stearinsäure | | | | | 2,5 |
| Myristylmyristat | | | | 1 | |
| Stearylalkohol | | | | 5 | |
| Cetearylalkohol | 3 | | 2 | | 1 |
| Cetylalkohol | | 1 | | | |
| Hydrierte Kokosfettglyceride | 1 | | | | 1 |
| Sheabutter | 2 | | | | |
| C12-15 Alkylbenzoat | 2 | 2 | | 2 | |
| Butylenglycol Dicaprylat/Dicaprat | | | | | 2 |
| Caprylsäure/Caprinsäure Triglycerid | 1 | | | 3 | |
| Hydriertes Polydecen | | | | 1 | |
| Ethylhexylkokosfettsäureester | | | | | 2 |
| Octyldodecanol | | | 1 | | 1 |
| Octamethyltetrasiloxan | 4 | | | | 2 |
| Dimethylpolysiloxan | | | | | 1 |
| Dicaprylylether | | | 2 | | |
| Mineralöl | | | 1 | | |
| Milchsäure | 1 | 2 | 0,5 | 2 | 0,5 |
| Glycolsäure | | | 1,5 | 2 | 0,5 |
| Salicylsäure | | | | | 1 |

| **Beispiel Nummer** | **6** | **7** | **8** | **9** | **10** |
|---|---|---|---|---|---|
| | | | | | |
| Citronensäure | | 1 | 0,5 | | |
| 1,2- Decandiol | 0,1 | 1 | 0,2 | 1 | 0,5 |
| 1,2- Dodecandiol | | | 0,2 | | |
| Tocopherylacetat | | | | | 0,05 |
| Trinatrium EDTA | | | 0,2 | 0,1 | |
| Iminodisuccinat | 0,2 | | | | 0,1 |
| Phenoxyethanol | 0,5 | 0,4 | 0,5 | | 0,3 |
| p-Hydroxybenzoesäurealkylester | 0,1 | | | 0,4 | 0,6 |
| Hexamidindiisethionat | | | 0,1 | | |
| Diazolidinylharnstoff | | 0,2 | | 0,1 | |
| lodopropynylbutylcarbamat | | | 0,25 | | |
| Ethanol denaturiert | | 8 | | | 3 |
| 2-Ethylhexylglycerinether | | | | 0,4 | |
| Xanthan Gummi | | 0,1 | | | |
| Polyacrylsäure | 0,2 | | 0,1 | | 0,1 |
| Polyacrylamid | | 0,2 | | | |
| Glycerin | 10 | 5 | 6 | 4 | 7 |
| Butylenglycol | | | | 2 | |
| wasser- und/oder öllösliche Farbstoffe | | | | | 0,1 |
| Additive (Distärkephosphat, SiO₂, Talkum, BHT Aluminiumstearat) | 0.03 | 0,1 | 0,05 | 3 | 1 |
| Parfüm | q.s. | q.s. | q.s. | q.s. | q.s. |
| Natriumhydroxid ad pH > 4,0 | q.s. | q.s. | q.s. | q.s. | q.s. |
| Wasser | Ad 100 | Ad 100 | Ad 100 | Ad 100 | Ad 100 |

### Beispielrezepturen Foundations

| **Inhaltsstoffe** | **1** | **2** | **3** | **4** |
|---|---|---|---|---|
| PEG-30-stearat | 1,5 | | | |
| Gylcerylstearat | 0,5 | | | 2 |
| Ceteareth-20 | 1 | | | |
| Polyglyceryl-3 Methylglucose stearat | | | 3,5 | |
| Sorbinsäurestearat | | | 1,5 | |
| Stearinsäure | 2 | | | 1,8 |
| Glycerylstearatcitrat | | 3,5 | | |
| Cetylalkohol | 0,5 | 0,75 | 1,0 | |
| Lecithin | | | | 0,5 |
| Veegum K = Mg-Al-Silicate | 0,8 | | | 1 |
| Xanthan Gum | 0,2 | | | 0,3 |
| Carbomer | | 0,2 | | |
| Hydroxyethylcellulose | 0,2 | | 0,1 | |
| Caprylsäure / Caprinsäure Triglycerid | | 2 | 3 | 2 |
| Dicaprylylether | | 2 | 3 | 3 |
| Octyldodecanol | | | 3 | |
| Dimethicon | 3 | | 3 | 3 |
| Cyclomethicon | | 4 | 3 | 2 |
| C12-C15 Alkyl Benzoate | 2 | | | |
| Cetearyloctanoat | 2 | | | |
| Squalan | 1 | | | 6 |
| Isopropylpalmitat | 1 | | | 2 |
| PPG -15 Stearylether | 2 | 3 | | |
| Hydrierete Kokos-Glyceride | 2 | | | |
| Stearyldimethicon | 9 | | | |
| Acetyliertes Lanolinöl | 2 | 0,2 | | |
| Milchsäure | 2 | 1 | 1 | |
| Glycolsäure | | | | 2 |
| Salicylsäure | | | 0,5 | |
| Citronensäure | | | 0,5 | 1 |
| 1,2- Decandiol | 0,2 | 1 | 0,4 | 2 |
| 1,2- Dodecandiol | | | 0,2 | |
| Retinol | 0,1 | | | |
| Burnet- Extrakt | | 0,025 | | |
| Silikon | | 0,8 | | |
| Nylon-12 | | | 5 | |
| Lauroyllysin | 0,5 | | 0,5 | |
| Kaolin | 0,5 | | 1 | 2 |
| Natrium Stärke Octenylsuccinat | | 1 | | 1,5 |
| Eisenoxide | 1,2 | 2,4 | 2,6 | 3 |
| Titandioxid | 3,8 | 5,6 | 4,5 | 6,5 |
| Interferenz Pigmente | 0,2 | | | 0,5 |
| Pigment Low Lustre | 0,2 | | | 0,2 |
| Polymethylsilsesquioxan | | 2 | | |
| EDTA | 0,1 | 0,6 | 1 | 1 |
| Glycerin | 2 | 5 | 5 | 10 |
| Phenoxyethanol und Paraben | 1 | 0,5 | | 1 |
| Imidazonidinylharnstoff | 0,3 | 0,3 | | 0,25 |
| Natriumhydroxid ad pH > 4,0 | q.s. | q.s. | q.s. | q.s. |
| Parfum, Antioxidantien | q.s. | q.s. | q.s. | q.s. |
| Wasser | ad 100 | ad 100 | ad 100 | ad 100 |

## Patentansprüche

1. Kosmetische und/oder dermatologische Zubereitung enthaltend eine Wirkstoffkombination aus
(a) einer oder mehreren α - und/oder β-Hydroxysäuren,
(b) einem oder mehreren Alkan-1,2-diolen,
neben gegebenenfalls weiteren kosmetischen und/oder dermatologischen Wirk-, Hilfs- und Zusatzstoffen, **dadurch gekennzeichnet, dass** das Verhältnis der Gesamtmenge an α-und/oder β-Hydroxysäuren zur Gesamtmenge an Alkan-1,2-diolen von 1:1 bis 1:0,2 beträgt mit der Maßgabe beträgt, dass der pH-Wert der kosmetischen und/oder dermatologischen Zubereitung größer 4,5 ist.

2. Kosmetische und/oder dermatologische Zubereitung nach Anspruch 1 zur Verwendung zur Prophylaxe und/oder Behandlung von Akne.

3. Kosmetische und/oder dermatologische Zubereitung nach Anspruch 1 zur Verwendung gegen Propionibakterium acnes.

4. Verwendung einer Zubereitung nach Anspruch 1
(i) zur Herstellung eines Medikamentes zur Prophylaxe und/oder Behandlung von Akne, oder
(ii) zur nichttherapeutischen Prophylaxe und/oder Behandlung von Akne.

5. Verwendung einer Zubereitung nach Anspruch 1
(i) zur Herstellung eines Medikamentes gegen Propionibakterium acnes, oder
(ii) zur nicht-therapeutische Prophylaxe und/oder Behandlung gegen Propionibakterium acnes.

6. Zubereitung nach einem der Ansprüche 1 bis 3 oder Verwendung nach einem der Ansprüche 4 bis 5, **dadurch gekennzeichnet, dass** als α - und/oder β-Hydroxysäure eine α -Hydroxysäure eingesetzt wird.

7. Zubereitung nach einem der Ansprüche 1 bis 3 oder Verwendung nach einem der Ansprüche 4 bis 5, **dadurch gekennzeichnet, dass** als Hydroxysäure Milchsäure eingesetzt wird.

8. Zubereitung nach einem der Ansprüche 1 bis 3 oder Verwendung nach einem der Ansprüche 4 bis 5, **dadurch gekennzeichnet, dass** als Alkan-1,2-diol Decan-1,2-diol eingesetzt wird.

9. Zubereitung nach einem der Ansprüche 1 bis 3 oder Verwendung nach einem der Ansprüche 4 bis 5, **dadurch gekennzeichnet, dass** eine oder mehrere α - und/oder β-Hydroxysäuren in einer Gesamtkonzentration von 0,1 bis 10 Gewichts -% und ein oder mehrere Alkan-1 ,2-diole in einer Gesamtkonzentration von 0,1 bis 5 Gewichts-%, jeweils bezogen auf das Gesamtgewicht der Zubereitung eingesetzt werden.

## Claims

1. Cosmetic and/or dermatologic preparation comprising an active agent combination of
(a) one or more α- and/or β-hydroxy acids,
(b) one or more alkane-1,2-diols,
besides further optional cosmetic and/or dermatologic active agents, excipients and additives, **characterized in that** the ratio of the total amount of α- and/or β-hydroxy acids to the total amount of alkane-1,2-diols ranges from 1:1 to 1:0.2 with the proviso that the pH value of the cosmetic and/or dermatologic preparation is greater 4.5.

2. Cosmetic and/or dermatologic preparation according to claim 1 for use in prophylaxis and/or treatment of acne.

3. Cosmetic and/or dermatologic preparation according to claim 1 for use against propionibacterium acnes.

4. Use of a composition according to claim 1
(i) for preparation of a medicament for prophylaxis and/or treatment of acne, or
(ii) for non-therapeutic prophylaxis and/or treatment of acne.

5. Use of a composition according to claim 1
(i) for preparation of a medicament against propionibacterium acnes, or
(ii) for non-therapeutic prophylaxis and/or treatment against propionibacterium acnes.

6. Preparation according to one of claims 1 to 3 or use according one of claims 4 to 5, **characterized in that** an α-hydroxy acid is used as α- and/or β-hydroxy acid.

7. Preparation according to one of claims 1 to 3 or use according to one of claims 4 to 5, **characterized in that** hydroxy lactic acid is used as α- and/or β-hydroxy acid.

8. Preparation according to one of claims 1 to 3 or use according one of claims 4 to 5, **characterized in that** decan-1,2-diol is used as alkane-1,2-diol.

9. Preparation according to one of claims 1 to 3 or use according one of claims 4 to 5, **characterized in that** one or more α- and/or β-hydroxy acids are used in a total concentration from 0.1 to 10 weight-% and one or more alkane-1,2-diols in a total concentration from 0.1 to 5 weight-%, in each case with respect to the total weight of the preparation.

## Revendications

1. Préparation cosmétique et/ou dermatologique contenant une combinaison de principes actifs de
(a) un ou plusieurs α- et/ou β-hydroxy-acides,
(b) un ou plusieurs alcane-1,2-diols,
outre, le cas échéant, d'autres principes actifs, excipients et additifs cosmétiques et/ou dermatologiques, **caractérisée par le fait que** le rapport de la quantité totale d'α- et/ou de β-hydroxy-acides sur la quantité totale d'alcane-1,2-diols est compris entre 1 : 1 et 1 : 0,2, sous réserve que la valeur pH de la préparation cosmétique et/ou dermatologique soit supérieure à 4,5.

2. Préparation cosmétique et/ou dermatologique selon la revendication 1, pour l'utilisation dans la prophylaxie et/ou le traitement de l'acné.

3. Préparation cosmétique et/ou dermatologique selon la revendication 1, pour l'utilisation contre Propionibacterium acnes.

4. Utilisation d'une préparation selon la revendication 1,
(i) pour la préparation d'un médicament pour la prophylaxie et/ou le traitement de l'acné ou
(ii) pour la prophylaxie et/ou le traitement non-thérapeutique de l'acné.

5. Utilisation d'une préparation selon la revendication 1,
(i) pour la préparation d'un médicament contre Propionibacterium acnes ou
(ii) pour la prophylaxie et/ou le traitement non-thérapeutique contre Propionibacterium acnes.

6. Préparation selon l'une quelconque des revendications 1 à 3 ou utilisation selon l'une quelconque des revendications 4 à 5, **caractérisée par le fait que** l'on utilise un α-hydroxy-acide comme α- et/ou β-hydroxy-acide.

7. Préparation selon l'une quelconque des revendications 1 à 3 ou utilisation selon l'une quelconque des revendications 4 à 5, **caractérisée par le fait que** l'on utilise l'acide lactique comme hydroxy-acide.

8. Préparation selon l'une quelconque des revendications 1 à 3 ou utilisation selon l'une quelconque des revendications 4 à 5, **caractérisée par le fait que** l'on utilise le décane-1,2-diol comme alcane-1,2-diol.

9. Préparation selon l'une quelconque des revendications 1 à 3 ou utilisation selon l'une quelconque des revendications 4 à 5, **caractérisée par le fait que** l'on utilise un ou plusieurs α- et/ou β-hydroxy-acides en une concentration totale comprise entre 0,1 et 10 % en poids et un ou plusieurs alcane-1,2-diols en une concentration totale comprise entre 0,1 et 5 % en poids, respectivement par rapport au poids total de la préparation.
